# FASCICULE DE BREVET EUROPEEN

(11) **EP 0 441 992 B1**
(45) Date de publication et mention de la délivrance du brevet: **18.01.1995**
(21) Numéro de dépôt: 90102667.4
(22) Date de dépôt: 12.02.1990
(51) Int. Cl.: C11B 7/00

(54) **Oléine de sal, procédé de préparation et compositions cosmétiques la contenant**
Verfahren zur Herstellung von Salolein und kosmetische Zusammensetzungen damit
Process for preparing salolein and cosmetic compositious therewith

(43) Date de publication de la demande: 21.08.1991
(73) Titulaire: SOCIETE DES PRODUITS NESTLE S.A., 1800 Vevey (CH)
(72) Inventeur: Mottier, Line, CH-1083 Mezières (CH); Viret, Jean-Louis, CH-1817 Brent (CH); Wille, Hans-Jürgen, CH-1844 Villeneuve (CH)

(56) Documents cités:
- GB-A- 2 109 233
- GB-A- 2 177 107
- JOURNAL OF THE AMERICAN OIL CHEMISTS' SOCIETY, vol. 65, no. 7, juillet 1985,pages 1126-1130, Champaign, Illinois, US; S.Y. REDDY et al.: "Effect oftriglycerides containing 9,10-dihydroxystearic acid on the solidificationproperties of Sal (shorea robusta) fat"

## Description

La présente invention a pour objet une oléine de sal comme constituant de la phase grasse de compositions cosmétiques.

Les compositions cosmétiques sous forme d'émulsion contiennent des corps gras susceptibles d'apporter les propriétés souhaitées d'application sur la peau, c'est-à-dire essentiellement des propriétés adoucissantes, lubrifiantes, nourrissantes et protectrices permettant de maintenir la souplesse de la peau et de la protéger des agressions atmosphériques.

Parmi les graisses d'origine végétales utilisables comme ingrédients des phases grasses on a proposé la graisse de sal provenant des graines des fruits de l'arbre Shorea robust. Selon le brevet des Etats-Unis No 4,534,981, par exemple, la graisse de sal apporte notamment les qualités requises d'assouplissement de la peau liées à un effet adoucissant. Cependant, du point de vue des propriétés physiques, cette graisse présente l'inconvénient de modifier négativement la cristallisation de la phase grasse des émulsions qui la contiennent, ce qui a pour effet d'écourter leur stabilité dans le temps et de plus de donner a ces émulsions un aspect moins attrayant en diminuant leur finesse et leur brillant.

On connaît des procédés de fractionnement par solvant de la graisse de sal en stéarine et oléine. Par exemple, la demande de brevet publiée WO 83.00 418 mentionne l'utilisation de l'acétone ou de 2-nitropropane; la demande de brevet japonais JP 81.127,694 a trait au fractionnement de la graisse de sal à l'aide de l'hexane, en deux étapes. Selon ces procédés, l'oléine obtenue est solide ou semi-solide à la température ambiante, autour de 20°C. L'invention a pour but de fournir un corps gras sous forme d'oléine de sal qui, tout en ayant les propriétés cosmétiques propres à la graisse de sal, n'a pas ses propriétés physiques préjudiciables à la qualité des émulsions.

L'invention concerne donc une oléine de sal caractérisée par le fait qu'elle se présente sous la forme d'un liquide limpide à 20°C et que sa teneur en matières grasses solides à 10°C est inférieure à 4,5% en poids.

L'invention concerne également un procédé de préparation de l'oléine de sal précédente, caractérisé par le fait:
- que l'on met la graisse de sal à une température supérieure à 20°C en présence de n-hexane dans les proportions graisse de sal: n-hexane de 1:3 à 1:6 en poids:volume,
- que l'on refroidit le mélange jusqu'à 5-12°C,
- que l'on ensemence le mélange ainsi refroidi avec 0,05 à 0,5% en poids de stéarine de sal,
- que l'on brasse lentement le mélange tout en le refroidissant jusqu'à une température inférieure à - 5°C par paliers ou progressivement et que l'on maintienne le mélange pendant 1 à 5h à cette température,
- que l'on sépare une fraction solide à une température inférieure à -5°C et que l'on recueille une fraction liquide qui constitue l'oléine, puis que l'on élimine le n-hexane de cette fraction liquide.

Pour mettre en oeuvre le procédé selon l'invention, on utilise de préférence la graisse de sal raffinée par les méthodes usuelles de démucilagination, neutralisation, blanchiment et désodorisation. On peut également utiliser la graisse semi-raffinée, c'est-à-dire traitée jusqu'au blanchiment, puis réaliser la désodorisation de l'oléine après le fractionnement.

On procède alors au fractionnement par le n-hexane par charges ou en semi-continu.

Celui-ci consiste à dissoudre la graisse dans une quantité suffisante de n-hexane, de préférence dans un rapport graisse de sal: hexane d'environ 1:4 en poids: volume, à une température de 20 à 60°C, de préférence à environ 20°C.

On refroidit ensuite la solution, par exemple dans un cristalliseur thermostaté, progressivement jusqu'à la température d'ensemencement, soit de préférence à 5-12°C avec ou sans brassage.

En variante, on peut mélanger la graisse de sal fondue par exemple à 40-60°C avec le n-hexane refroidi, par exemple à une température de -5°C à 0°C.

Dans le but d'obtenir une cristallisation dirigée, c'est-à-dire que les cristaux soient relativement homogènes, on ensemence la solution précédente avec des cristaux de stéarine de sal. Les cristaux en question peuvent être obtenues la première fois par une mise en oeuvre du procédé sans ensemencement et par la suite, de préférence à partir d'une charge précédente. Le taux d'ensemencement est de 0,05 à 0,5% et de préférence environ 0,1% en poids.

Par la suite, on refroidit progressivement la dispersion sous brassage lent, de préférence avec une vitesse de refroidissement de 5 à 10°C/h, jusqu'à une température du fluide de refroidissement de -7 à -20°C, par exemple à -9°C, puis on maintient cette température pendant 2 à 5h, de préférence pendant environ 4h sous brassage lent.

Il se forme des cristaux nodulaires denses que l'on peut séparer par filtration en utilisant par exemple un dispositif de filtration sous vide ou bien encore par décantation ou essorage. On recueille la fraction liquide.

On peut laver les cristaux avec du n-hexane refroidi, de préférence à environ -10°C et recueillir les eaux de lavage que l'on peut joindre à la fraction liquide précédente.

Ensuite, on élimine le n-hexane des liquides contenant l'oléine, par exemple par évaporation sous vide. De préférence, on traite les liquides désolvantisés par entraînement à la vapeur sous vide dans des conditions ne provoquant pas d'isomérisation, de préférence sous une pression de 1 à 4 mbar et à une température inférieure à 220°C, par exemple de l'ordre de 200°C de manière à éliminer toute trace de n-hexane.

L'invention concerne également une composition cosmétique contenant 2 à 80% en poids d'oléine de sal.

La composition cosmétique peut être aqueuse ou anhydre. Une composition aqueuse selon l'invention peut se présenter sous forme d'émulsion eau-dans-l'huile ou huile-dans-l'eau, la concentration en oléine de sal étant de préférence 4 à 20% en poids. Ce peut être par exemple un gel hydratant ou démaquillant, un lait, une crème de soins ou solaire, un fond de teint. Dans une telle composition, la phase grasse peut contenir d'autres huiles, animales, végétales, minérales ou synthétiques. Elle peut contenir également des cires, des alcools à longue chaîne, des agents épaississants, gélifiants. Lorsqu'il s'agit d'une émulsion, une composition cosmétique contient 1 à 20% en poids d'un agent émulsifiant.

Dans une composition cosmétique anhydre, la phase grasse peut contenir de 10 à 80% en poids, et de préférence de 10 à 40% en poids d'oléine de sal par rapport au poids total de la composition. En outre, elle peut contenir d'autres huiles et une proportion relativement élevée, par exemple de 5 à 30% en poids de cires. Elle peut se présenter par exemple sous forme d'huile antisolaire (auquel cas elle contient un filtre solaire absorbant les rayons ultra-violets), de baume anhydre, de rouge à lèvres.

Les compositions selon l'invention peuvent contenir en outre divers additifs, notamment des agents colorants, des parfums, des conservateurs, des filtres UV, des agents nacrants et des charges minérales ou organiques. Elles contiennent avantageusement des antioxydants, à raison de 0,02 à 0,2% en poids.

Les exemples ci-après illustrent l'invention. Dans ceux-ci, les parties et pourcentages sont pondéraux sauf indication contraire.

### Exemple 1

On utilise une graisse de sal raffinée dont la composition en acides gras déterminée par analyse chromatographique des esters méthyliques est la suivante:

| | % |
|---|---|
| C 16:0 | 5,1 |
| C 18:0 | 43,6 |
| C 18:1 | 39,4 |
| C 18:2 | 1,7 |
| C 18:3α | 0,6 |
| C 20:0 | 7,9 |
| C 20:1 | 1,2 |
| autres | 0,5 |

Dans un réacteur de 100 l chauffé à 52°C on mélange sous agitation 15 kg de graisse de sal et 60 l de n-hexane jusqu'à dissolution complète. On refroidit ensuite le mélange à 8°C, puis on y ajoute 15 g de stéarine de sal obtenue de la même manière que selon cet exemple en omettant simplement l'ensemencement. On refroidit alors le mélange par paliers, d'abord à 0°C en brassant lentement en 3h, puis à -6°C en brassant lentement en 1h. On filtre la suspension de cristaux formée à l'aide d'un filtre refroidi à 0°C. Après lavage des cristaux avec 10 l de n-hexane refroidi à -10°C, on joint les eaux de lavage au filtrat, puis on évapore le n-hexane de la solution dans un évaporateur rotatif sous vide. On recueille ainsi 5,16 kg d'oléine de sal dont la composition en acides gras, déterminée par chromatographie est la suivante:

| | % |
|---|---|
| C 16:0 | 6,2 |
| C 18:0 | 26,7 |
| C 18:1 | 54,7 |
| C 18:2 | 4,1 |
| C 18:3α | 1.2 |
| C 20:0 | 5,0 |
| C 20:1 | 1,8 |
| autres | 0,3 |

On détermine l'indice de graisse solide ("solid fat content", SFC) à partir de la courbe de fusion de la matière grasse par RMN (résonance magnétique nucléaire du proton) pulsée. C'est le pourcentage de graisse solide existant dans le corps gras partiellement fondu à une température donnée. Les résultats sont les suivants:

| | SFC (%) à la température (°C) de | |
|---|---|---|
| | 10 | 20 |
| Graisse de sal | 77 | 61 |
| Oléine de sal | 3 | 0 |

On voit que l'oléine de sal obtenue selon l'invention est parfaitement liquide à la température d'entreposage ordinaire, autour de 20°C.

### Exemple 2

On dissout 100 g de la graisse de sal raffinée utilisée comme matière première dans l'exemple 1 dans un ballon dans 400 ml de n-hexane sous agitation lente. Le ballon est maintenu à 20,8°C. Après dissolution, on refroidit progressivement le mélange en maintenant le ballon dans un fluide frigorifique tout en brassant à une vitesse de 100 t/min. On atteint la température de -9°C au niveau du fluide frigorifique en 3h30. Lorsque la température de 5°C est atteinte, on ensemence la solution avec 0,1% de stéarine de sal. On maintient la température finale de -9°C pendant 2h sous brassage lent, puis on filtre les cristaux apparus sur filtre Büchner refroidi à -10°C. Après lavage avec du n-hexane refroidi à -10°C, on évapore le solvant dans un évaporateur rotatif sous vide. On obtient ainsi 33,8 g d'oléine de sal qui sert de référence dans les exemples comparatifs ci-après.

### Exemples comparatifs 1-2

1. A titre de comparaison, on effectue le fractionnement même matière première:
a) selon la demande de brevet japonais JP 81.127.694
b) selon l'exemple 1 de la demande de brevet publiée WO 83.00.418.
Dans le tableau I ci-après, on indique les valeurs de SFC, le rendement, l'aspect à 20°C et la composition en acides gras des oléines obtenues.

**Tableau I**

| SFC(%)à | Comparaison a) | Comparaison b) | Référence |
|---|---|---|---|
| 0°C | 41,5 | 53,6 | 27,5 |
| 10°C | 14 | 43,6 | 2,7 |
| 20°C | 1,5 | 21 | 0 |
| 30°C | 0,2 | 1,8 | 0 |
| Rendement (%) | 38,3 | 53,1 | 34,4 |
| Aspect à 20°C | semi-solide | solide | liquide limpide |

| Composition en acides gras principaux (%) | Comparaison a) | Comparaison b) | Référence |
|---|---|---|---|
| C 16:0 | 6,7 | 7,5 | 6 |
| C 18:0 | 29,9 | 34,7 | 23,2 |
| C 18:1 | 51,4 | 46,6 | 58,2 |
| C 18:2 | 3,7 | 3 | 5,1 |
| autres | 8,3 | 8,2 | 7,5 |

2. On effectue le fractionnement de la même oléine de sal dans des conditions optimisées avec les solvants usuels acétone (c) et d)) et isopropanol (e), f) et g)). Ces conditions sont indiquées dans le tableau II ci-après. On détermine chaque fois le SFC à différentes températures et le rendement en oléine.

**Tableau II**

| Essai | Conditions de fractionnement | | | SFC (%) | | | Rendement (%) |
|---|---|---|---|---|---|---|---|
| | Rapport graisse/solvant | Température finale (°C) | Durée à la température finale (h) | A la température de (°C) | | | |
| | | | | 0 | 10 | 20 | |
| Référence | 1/4 | -9 | 2 | 33,5 | 3,4 | 0 | 33,1 |
| c) | 1/3 | 0 | 3 | 23,2 | 1,3 | 0,1 | 18 |
| d) | 1/4 | 0 | 3 | 27,2 | 2,4 | 0,5 | 21,1 |
| e) | 1/3 | 4 | 3 | 32,2 | 5,2 | 0,3 | 23,9 |
| f) | 1/3 | 15 | 2 | 32 | 9,4 | 4,1 | 23,3 |
| g) | 1/3 | 10 | 2 | 28,2 | 6,5 | 3,3 | 18,5 |

Les résultats des essais comparatifs ci-dessus (Tab. I) montrent sans équivoque que seule l'oléine obtenue selon l'invention avec le n-hexane ne conduit pas à la formation de cristaux à la température ambiante (SFC 0% à 20°C). Les autres solvants utilisés couramment dans le fractionnement des graisses (Tab. II) conduisent à un rendement en oléine inférieur.

### Exemples 3-15

Ces exemples concernent l'application dermo-cosmétique de l'oléine de sal selon l'invention.
Dans ces exemples, la nomenclature utilisée est celle de la "Cosmetic, Toiletery and Flagrance Association, Inc. Washington DC" (CTFA).

Pour fabriquer les émulsions, on mélange les composants de la phase lipidique A et on la chauffe à 70°C. On prépare la phase aqueuse B en mélangeant ses composants et on la chauffe à 70°C. On ajoute la phase lipidique A à la phase aqueuse B à 70°C en brassant à vitesse moyenne. On homogénéise le mélange des deux phases puis on le brasse à 100 t/min et on le laisse refroidir à 45-50°C pour les émulsions eau-dans-l'huile et à 35-40°C pour les émulsions huile-dans-l'eau.

A cette température, on ajoute le cas échéant les additifs C, puis on continue à refroidir jusqu'à la température ambiante en brassant lentement et on arrête le brassage lorsque le produit est semi-fluide.

Les produits anhydres sont obtenus de la même manière, mais sans homogénéisation par mélange à chaud, puis refroidissement progressif sous brassage lent.

### 3. Gel démaquillant (émulsion huile-dans-l'eau)

| | % | |
|---|---|---|
| Phase lipidique A | | 20,55 |
| Peg 8-C12-C20 alkyl ester (Esters d'alcools gras en C12-C20 polyoxyéthylénés et polyglycérolés) | 4,0 | |
| oléine de sal | 5,0 | |
| 4-Ethyl-laurate | 10 | |
| Nonoxynol-10 (polyglycoléther) | 1,5 | |
| Triéthylcitrate, butylhydroxyanisole (BHA) et tocophérol | 0,05 | |
| Phase aqueuse B | | 74,87 |
| Carbomer 940 (polymère d'acide acrylique polyréticulé, dispersion dans l'eau à 2% | 40 | |
| Eau | 34,87 | |
| Additifs C | | 4,58 |
| Glucamine, solution aqueuse à 20% | 4,5 | |
| Méthylchlorothiazolinone et méthylisothiazolinone | 0,08 | |
| | | 1̅0̅0̅ |

### 4. Lait démaquillant (émulsion huile-dans-l'eau)

| | % | |
|---|---|---|
| Phase lipidique A | | 21,55 |
| Oléine de sal | 7 | |
| 2-éthyl-hexyl-2-éthyl hexanoate | 3 | |
| Triglycéride d'acides gras en C10-C18 | 4 | |
| Huile de paraffine | 3 | |
| Stéarate de glycérol | 3 | |
| Acide stéarique | 1,5 | |
| Tocophérol, BHA et triéthyl citrate | 0,05 | |
| Phase aqueuse B | | 48,07 |
| Eau | 47,88 | |
| Tétrahydroxypropyl-éthylenediamine | 0,14 | |
| Ethylènediaminetétraacétate (EDTA) disodique | 0,05 | |
| Additifs C | | 30,38 |
| Hydroxyéthylcellulose, solution aqueuse à 2% | 30 | |
| Méthylchlorothiazolinone et Méthylisothiazolinone | 0,08 | |
| Parfum | 0,3 | |
| | | 1̅0̅0̅ |

### 5. Crème hydratante (émulsion huile-dans-l'eau)

| | % | |
|---|---|---|
| Phase lipidique A | | 26,05 |
| Peg 8-C12-C18 alkyl ester (Esters d'alcools gras en C12-C18 polyoxyéthylénés et polyglycérolés) | 10 | |
| Oléine de sal | 7 | |
| Laurate d'isodécyle | 5 | |
| Alcool cétéarylique | 4 | |
| Tocophérol, BHA et Triéthyl citrate | 0,05 | |
| Phase aqueuse B | | 73,87 |
| Eau | 62,87 | |
| Alcools gras en C12-C18 polyoxyéthylénés et polyglycérolés | 2 | |
| Propylène glycole | 5 | |
| Panthénol | 2 | |
| Sodium PCA | 2 | |
| Additif C | | 0.08 |
| Méthylchlorothiazolinone et Méthylisothiazolinone | 0,08 | |
| | | 1̅0̅0̅ |

### 6. Crème démaquillante (émulsion huile-dans-l'eau)

| | % | |
|---|---|---|
| Phase lipidique A | | 24,55 |
| Oléine de sal | 7 | |
| 2-éthyl-hexyl-2-éthyl hexanoate | 3 | |
| Stéaréth-21 (émulsifiant non ionique) | 2 | |
| Alcool cétéarylique | 1 | |
| Triglycéride d'acides gras en C10-C18 | 4 | |
| Huile de paraffine | 3 | |
| Stéarate de glycérol | 3 | |
| Acide stéarique | 1,5 | |
| Tocophérol, BHA et triéthyl citrate | 0,05 | |
| Phase aqueuse B | | 45,07 |
| Eau | 44,88 | |
| Tétrahydroxypropyl-éthylenediamine | 0,14 | |
| Ethylènediaminetétraacétate (EDTA) disodique | 0,05 | |
| Additifs C | | 30,38 |
| Hydroxyéthylcellulose, dispersion aqueuse à 2% | 30 | |
| Méthylchlorothiazolinone et Méthylisothiazolinone | 0,08 | |
| Parfum | 0,30 | |
| | | 1̅0̅0̅ |

### 7. Lait au collagène (émulsion huile-dans-l'eau)

| | % | |
|---|---|---|
| Phase lipidique A | | 21,55 |
| Oléine de sal | 7 | |
| Triglycéride en C10-C18 | 4 | |
| 2-éthyl-hexyl-2-éthyl hexanoate | 3 | |
| Huile de paraffine | 3 | |
| Stéarate de glycérol | 3 | |
| Acide stéarique | 1,5 | |
| Tocophérol, triéthyl citrate et BHA | 0,05 | |
| Phase aqueuse B | | 45,07 |
| Eau | 43,62 | |
| Tétrahydroxypropyl-éthylenediamine | 1,4 | |
| Ethylènediaminetétraacétate (EDTA) disodique | 0,05 | |
| Additifs C | | 33,38 |
| Hydroxyéthylcellulose, suspension aqueuse à 2% | 30 | |
| Collagène | 3 | |
| Méthylchlorothiazolinone et Méthylisothiazolinone | 0,08 | |
| Parfum | 0,30 | |
| | | 1̅0̅0̅ |

### 8. Lait hydratant (émulsion huile-dans-l'eau)

| | % | |
|---|---|---|
| Phase lipidique A | | 27,05 |
| Isocétéareth-10 stéarate | 10 | |
| Oléine de sal | 7 | |
| Laurate d'isodécyle | 5 | |
| Alcool cétéarylique | 2 | |
| Stéarate de glycérol | 3 | |
| Tocophérol, BHA et triéthyl citrate | 0,05 | |
| Phase aqueuse B | | 72,67 |
| Eau | 48,67 | |
| Hydroxyéthylcellulose, suspension aqueuse à 2% | 20 | |
| Panthénol | 2 | |
| Sodium PCA | 2 | |
| Additifs C | | 0,28 |
| Parfum | 0,1 | |
| Tétrasodium étidronate | 0,1 | |
| Méthylchlorothiazolinone et Méthylisothiazolinone | 0,08 | |
| | | 1̅0̅0̅ |

### 9. Emulsion teintée hydratante (fond-de-teint, huile-dans-l'eau)

| | | % | |
|---|---|---|---|
| Phase lipidique A | | | 18,95 |
| Isocéteth-10-stéarate et isocétéareth-10 stéarate | | 7 | |
| Oléine de sal | | 4,9 | |
| Laurate d'isodécyle | | 3,5 | |
| Alcool cétéarylique | | 1,4 | |
| Stéarate de glycérol | | 2,1 | |
| Tocophérol, BHA et triéthyl citrate | | 0,05 | |
| Phase aqueuse B | | | 50,77 |
| Eau | | 32,77 | |
| Hydroxyéthylcellulose, suspension aqueuse à 2% | | 14 | |
| Panthénol | | 2 | |
| Sodium PCA | | 2 | |
| Additifs C | | | 30,28 |
| Pigments concentrés | blanc | 22,7 | |
| | jaune | 4,8 | |
| | rouge | 1,5 | |
| | brun à 40% de pigment, le solde étant de la glycérine, du sorbitol et du polysorbate 20 | 1 | |
| Parfum | | 0,1 | |
| Tétrasodium étidronate | | 0,1 | |
| Méthylchlorothiazolinone et Méthylisothiazolinone | | 0,08 | |
| | | | 1̅0̅0̅ |

### 10. Crème de soins (émulsion eau-dans-l'huile)

| | % | |
|---|---|---|
| Phase lipidique A | | 39 |
| Peg-1 glycérol sorbitane oléostéarate et cire de paraffine | 12 | |
| Huile de paraffine | 13 | |
| Oléine de sal | 8 | |
| Triglycérides d'acides caprylique et caprique | 5 | |
| 2-Phénoxyéthanol, méthylparabène, éthylparabène, propylparabène et butylparabène | 1 | |
| Phase aqueuse B | | 61 |
| Eau | 58,3 | |
| Sulfate de magnésium heptahydraté | 0,7 | |
| Glycérine | 2 | |
| | | 1̅0̅0̅ |

### 11. Crème solaire (émulsion eau-dans-l'huile)

| | % | |
|---|---|---|
| Phase lipidique A | | 39 |
| Peg-1 glycérol sorbitane oléostéarate et cire de paraffine | 12 | |
| Huile de paraffine | 11 | |
| Oléine de sal | 8 | |
| Triglycérides d'acides caprylique et caprique | 5 | |
| Octyl-méthoxycinnamate | 2 | |
| 2-Phénoxyéthanol, méthylparabène, éthylparabène, propylparabène et butylparabène | 1 | |
| Phase aqueuse B | | 61 |
| Eau | 58,3 | |
| Sulfate de magnésium heptahydraté | 0,7 | |
| Glycérine | 2 | |
| | | 1̅0̅0̅ |

### 12. Huile pour le visage et le corps (anhydre)

| | % |
|---|---|
| Huile de paraffine | 56,85 |
| Oléine de sal | 10 |
| Octanoate d'octyle | 10 |
| Triglycérides en C10-C18 | 10 |
| Huile de silicone | 5 |
| Laurate d'isodécyle | 5 |
| Méthoxycinnamate d'octyle | 3 |
| Parfum | 0,1 |
| Tocophérol, triéthyl citrate et BHA | 0,05 |
| | 1̅0̅0̅ |

### 13. Baume anhydre

| | % |
|---|---|
| Paraffine | 4 |
| Ozokérite | 5 |
| 2-éthyl-hexyl-2-éthyl hexanoate | 45,6 |
| Oléine de sal | 40 |
| Isodécyle laurate | 5 |
| Antioxydant | 0,1 |
| Parfum | 0,3 |
| | 1̅0̅0̅ |

### 14. Gel démaquillant anhydre

| | % |
|---|---|
| Huile de paraffine | 45,6 |
| Oléine de sal | 30 |
| 2-éthyl-hexyl-2-éthyl hexanoate | 10 |
| Laurate d'isodécyle | 5 |
| Ozokérite | 5 |
| Paraffine | 4 |
| Tocophérol, BHA et Triéthyl citrate | 0,1 |
| Parfum | 0,3 |
| | 1̅0̅0̅ |

### 15. Rouge à lèvres (anhydre)

| | |
|---|---|
| Huile de ricin | 27,45 |
| Myristate d'isopropyle | 20 |
| Oléine de sal | 10,5 |
| Cire d'abeilles | 10,5 |
| Cire de candelilla | 7,5 |
| Ozokérite | 5,5 |
| Lanolate d'isopropyle | 5 |
| Colorants | 13,55 |
| | 1̅0̅0̅ |

Tous les produits cosmétiques des exemples 3-15 ont été testés et présentent une bonne stabilité à 20°C, 37°C et 47°C pendant 3 mois.

Ils ont de bonnes propriétés organoleptiques soit:
- les émulsions comme les produits anhydres sont homogènes, fins, lisses et brillants.
- Ils présentent un bon étalement sur la peau et une bonne pénétration cutanée. La peau a un bon toucher, est lisse, douce et soyeuse.

Pendant la fabrication, au cours des tests et analyses physico-chimiques, il n'y a pas eu de cristallisation de la phase lipidique avec l'oléine alors que l'utilisation de la graisse de sal conduit à des problèmes de stabilité dus à la cristallisation.

## Revendications

1. Oléine de sal, caractérisée par le fait qu'elle se présente sous la forme d'un liquide limpide à 20°C et que sa teneur en matières grasses solides à 10°C est inférieure à 4,5% en poids.

2. Procédé de préparation de l'oléine de sal selon la revendication 1, caractérisé par le fait:
- que l'on met la graisse de sal à une température supérieure à 20°C en présence de n-hexane dans les proportions graisse de sal:n-hexane de 1:3 à 1:6 en poids:volume,
- que l'on refroidit le mélange jusqu'à 5-12°C,
- que l'on ensemence le mélange ainsi refroidi avec 0,05 à 0,5% en poids de stéarine de sal,
- que l'on brasse lentement le mélange tout en le refroidissant jusqu'à une température inférieure à - 5°C par paliers ou progressivement et que l'on maintienne le mélange pendant 1 à 5h à cette température,
- que l'on sépare une fraction solide par filtration à une température inférieure à -5°C et que l'on recueille une fraction liquide qui constitue l'oléine, puis que l'on élimine le n-hexane de cette fraction liquide.

3. Procédé selon la revendication 2, caractérisé par le fait que le refroidissement est progressif et que la vitesse de refroidissement du mélange jusqu'à la température finale est de 5 à 10°C/h.

4. Composition cosmétique contenant une phase grasse caractérisée par le fait que ladite phase grasse contient une oléine de sal selon la revendication 1.

5. Composion selon la revendication 4, caractérisée par le fait que l'oléine de sal est présente à une concentration de 2 à 80% en poids de la composition.

6. Composition selon la revendication 4, caractérisée par le fait qu'elle se présente sous forme d'une émulsion eau-dans-l'huile ou huile-dans-l'eau, qu'elle contient 1 à 20% en poids d'un agent émulsifiant et que la phase grasse contient 4 à 20% en poids d'oléine de sal, par rapport au poids total de l'émulsion.

7. Composition selon la revendication 4, caractérisée par le fait qu'elle se présente sous forme anhydre et que la phase grasse contient 10 à 80% en poids d'oléine de sal, par rapport au poids total de la composition.

8. Composition selon l'une des revendications 4 à 7, caractérisé par le fait qu'elle contient 0,02 à 0,2% en poids d'antioxydant.

## Claims

1. Sal olein characterised in that it is in the form of a clear liquid at 20°C and in that its solid fat content at 10°C is less than 4.5% by weight.

2. Process for preparing sal olein according to Claim 1, characterised in that:
- sal fat is raised to a temperature above 20°C in the presence of n-hexane so that the proportion of sal fat to n-hexane is between 1:3 and 1:6 weight/volume,
- the mixture is cooled to 5-12°C,
- the mixture thus cooled is seeded with 0.05 to 0.5% by weight of sal stearin,
- the mixture is stirred slowly whilst cooling it to a temperature below -5°C in stages or progressively, and in that the mixture is held at this temperature for 1 to 5 hours,
- a solid fraction is separated by filtration at a temperature below -5°C and a liquid fraction is collected, which constitutes the olein, and then the n-hexane is eliminated from this liquid fraction.

3. Process according to Claim 2, characterised in that the cooling is progressive and the rate of cooling of the mixture to the final temperature is 5 to 10°C/h.

4. Cosmetic composition containing a fatty phase, characterised in that the said fatty phase contains a sal olein according to Claim 1.

5. Composition according to Claim 4, characterised in that the sal olein is present at a concentration of 2 to 8% by weight of the composition.

6. Composition according to Claim 4, characterised in that it is in the form of a water-in-oil or oil-in-water emulsion, in that it contains 1 to 20% by weight of an emulsifier and in that the fatty phase contains 4 to 20% by weight of sal olein with respect to the total weight of the emulsion.

7. Composition according to Claim 4, characterised in that it is in anhydrous form and in that the fatty phase contains 10 to 80% by weight of sal olein with respect to the total weight of the composition.

8. Composition according to one of Claims 4 to 7, characterised in that it contains 0.02 to 0.2% by weight of antioxidant.

## Patentansprüche

1. Salolein, dadurch gekennzeichnet, daß es in Form einer bei 20 °C klaren Flüssigkeit vorliegt und daß sein Gehalt an festen Fetten bei 10 °C unterhalb von 4,5 Gew.-% liegt.

2. Verfahren zur Herstellung eines Saloleins nach Anspruch 1, dadurch gekennzeichnet,
- daß man das Salfett bei einer Temperatur oberhalb von 20 °C mit n-Hexan in Verhältnissen Salfett : n-Hexan von 1:3 bis 1:6 Gewicht/Volumen zusammenbringt,
- daß man das Gemisch auf 5 bis 12 °C abkühlt,
- daß man das gekühlte Gemisch mit 0,05 bis 0,5 Gew.-% Salstearin animpft,
- daß man das gesamte Gemisch unter leichtem Rühren stufenweise oder kontinuierlich bis auf eine Temperatur unterhalb von -5 °C abkühlt und das Gemisch bei dieser Temperatur 1 bis 5 h hält,
- daß man durch Filtration bei einer Temperatur unterhalb von -5 °C eine feste Fraktion abtrennt und daß man eine flüssige Fraktion gewinnt, die das Olein bildet, wonach man das n-Hexan aus dieser flüssigen Fraktion abzieht.

3. Verfahren nach Anspruch 2, dadurch gekennzeichnet, daß die Abkühlung kontinuierlich ist und daß die Geschwindigkeit der Abkühlung der Mischung bis auf die Endtemperatur von 5 bis 10 °C/h beträgt.

4. Kosmetische Zusammensetzung, die eine Fettphase enthält, dadurch gekennzeichnet, daß diese Fettphase ein Salolein nach Anspruch 1 enthält.

5. Verfahren nach Anspruch 4, dadurch gekennzeichnet, daß das Salolein in einer Konzentration von 2 bis 80 Gew.-% der Zusammensetzung vorliegt.

6. Zusammensetzung nach Anspruch 4, dadurch gekennzeichnet, daß sie in Form einer Wasser-in-Öl- oder einer Öl-in-Wasser-Emulsion vorliegt, die 1 bis 20 Gew.-% eines Emulgators enthält und bei der die Fettphase 4 bis 20 Gew.-% Salolein, bezogen auf das Gesamtgewicht der Emulsion, enthält.

7. Zusammensetzung nach Anspruch 4, dadurch gekennzeichnet, daß sie in wasserfreier Form vorliegt und die Fettphase 10 bis 80 Gew.-% Salolein, bezogen auf das Gesamtgewicht der Zusammensetzung, enthält.

8. Zusammensetzung nach einem der Ansprüche 4 bis 7, dadurch gekennzeichnet, daß sie 0,02 bis 0,2 Gew.-% eines Antioxidans enthält.
